# EUROPEAN PATENT SPECIFICATION

(11) **EP 2 914 242 B1**
(45) Date of publication and mention of the grant of the patent: **13.12.2017**
(21) Application number: 13792719.0
(22) Date of filing: 31.10.2013
(51) Int. Cl.: A61K 8/97, A61Q 1/02, A61Q 1/06, A61Q 5/12, A61Q 19/00, A61Q 19/08

(54) **COSMETIC COMPOSITIONS CONTAINING FRACTIONS OF LINGONBERRY EXTRACTS**
KOSMETISCHE ZUSAMMENSETZUNGEN ENTHALTEND FRAKTIONEN VON PREISELBEERENEXTRAKTEN
COMPOSITIONS COSMÉTIQUES CONTENANT DES FRAGMENTS D'EXTRAITS D'AIRELLE ROUGE

(30) Priority: 01.11.2012 FI 20126144
(43) Date of publication of application: 09.09.2015
(73) Proprietor: Lumene Oy, 02780 Espoo (FI)
(72) Inventor: BACKMAN, Josefin, FI-10300 Karis (FI); ISOHANNI, Tiina, FI-02400 Kirkkonummi (FI); JAAKKOLA, Mari, FI-88600 Sotkamo (FI); MALINEN, Hanna-Liisa, FI-88600 Sotkamo (FI); MÄKI, Marianne, FI-88600 Sotkamo (FI); RÄTY, Jarkko, FI-88600 Sotkamo (FI); VIRTANEN, Vesa, FI-88600 Sotkamo (FI)
(74) Representative: Seppo Laine Oy
(86) International application number: PCT/FI2013/051030
(87) International publication number: WO 2014/068190

(56) References cited:
- WO-A1-2011/015706
- JP-A- 2000 256 176
- JP-A- 2002 363 057
- US-A1- 2005 175 762
- US-A1- 2009 011 056
- US-A1- 2010 297 272

## Description

### Field of the Invention

In the present invention side streams of berry industry are exploited, especially the press cake obtained when pressing juice from lingonberry. In specific, the dried fibrous part of the press cake, i.e. lingonberry fiber powder, is used as a raw material in producing ingredients which are useful in cosmetic compositions.

### Background of the Invention

Berries have had an important role in human diet for ages and nowadays also their health effects have been widely recognized. The health effects are often connected to the phenolic compounds found in abundance in berries. Many phenolic compounds are well known antioxidants but they also protect berries against hazards from their environment, e.g. from excess UV radiation of the sun or against attacks of viruses, bacteria and fungi. Also the colors of berries are influenced by the combinations of different phenolic compounds in each berry species. These compounds are thus mostly located on the skin layers of the berries.

Berry juice production is a common practice to utilize berries in an industrial scale. The juice is pressed from berries and the skins and seeds are left in the press residue, which is also called a press cake. The press cake is highly perishable material as such, but drying extends its usefulness and shelf life. A big challenge for the berry industry has been to find profitable use for this side stream, which may form up to 30% of the original weight of the berries. The seeds are to some extent used to make berry seed oils, and the fibrous berry skins as a berry fiber powder is used in food applications. Still plenty of press residue and hence significant amounts of berry phenolic compounds remain unutilized. New means to process and new applications to utilize this valuable material are needed.

Some phenolic compounds, e.g. quercetin and kaempferol, are chemically manufactured and commonly used ingredients in skin conditioning. However, a common trend is towards using natural ingredients in cosmetic products. Therefore, the cosmetic industry is constantly searching for new safe ingredients of plant origin. In addition, these ingredients should be produced cost effectively with minimal and simple processing and should maintain their activity in final products.

Quercetin has numerous advantageous characteristics, e.g. antioxidative and anti-inflammatory properties. Quercetin is categorized as a flavonol by structure. All flavonols have in common a 3-hydroxyflavone backbone, which can carry further phenolic OH groups in various positions. These compounds as well as phenolic compounds in general are present in many fruits, berries and vegetables and they mostly appear as conjugated glycosides, i.e. bound to various sugar molecules.

Lingonberries (*Vacciniun vitis-idaea*) are known to contain e.g. quercetin and kaempferol glycosides among other phenolic compounds. The abundance of kaempferol glycosides is significantly lower than that of quercetin glycosides (Ek et al., 2006 and USDA Database for the Flavonoid Content of Selected Foods, Release 3.0, 2011).

The phenolic composition of lingonberries has been widely studied but it is not yet characterized thoroughly. The amounts of different compounds are known to vary according to many factors, such as the growing environment. The conjugated structures also cause additional challenge to analyzing. The main classes of phenolic compounds found in lingonberries are hydroxybenzoic acids, hydroxycinnamic acids, anthocyanins, flavonols, flavan-3-ols and proanthocyanidins, as recently reviewed by Kylli (2011).

In patent literature various patent applications disclose methods for extracting, isolating and enriching phenolic compounds from plant material, e.g. berries. For instance, US patent application No. 2005/175762 describes extraction of lipophilic cranberry antioxidants such as quercetin, isoquercitrin, cinnamic acid, anthocyanin, flavonoid or proanthocyanidin using resin column fractionation with water/ethanol/methanol/acetone solvent system or chloroform: methanol:water partitioning from cranberry juice powder. From cranberry press cake the phenolic compounds are extracted by aqueous acetone. The resulting extract is further extracted with various organic solvents, e.g. butanol, chloroform, dichloromethane, ethyl acetate or ether. The organic phase from various extractions is evaporated and the remaining extracts are freeze-dried. Said methods, as most of the known methods use harmful organic solvents.

US patent No. 7,208,181 discloses a method for isolating polyphenolic compounds from fruits and berries. The method is based on subcritical water extraction (SWE) instead of organic solvent extraction. In SWE, however, the requirements of instrumentation and subsequent production costs are high.

US patent application No. 2009/011056 focuses on producing an extract enriched with anthocyanin, proanthocyanin and resveratrol from lingonberry fruit. Extracts are obtained by extracting the same batch of lingonberries twice. The first extraction is made using alcohol, aqueous alcohol, water, aqueous acid, aqueous enzyme-containing solution, or aqueous acid-alcohol solution. The extract is concentrated and purified, and the first extraction residue is re-extracted with ethyl acetate, benzene, toluene, chloroform, dichloromethane, n-hexane, acetone, methanol, ethanol, or a mixture thereof, or aqueous methanol or ethanol solution or aqueous solution containing cellulase or pectase enzymes. This second extract is also concentrated and purified. The final lingonberry compounds are obtained by mixing the first and the second concentrated and purified extracts in different ratios.

US patent application No. 2010/297272 also discloses methods, in which lingonberry compositions rich in anthocyanidin, procyanidin and resveratrol are obtained. Two extracts made of lingonberry fruits are mixed, but the solvents used and details in purification steps differ from those used in US 2009/011056. The methods in both of the applications, like many known fractionation methods for phenolic compounds, include purification steps where e.g. column purifications with chemical resins or membrane filtration techniques are used. These types of purification methods or using aqueous solvents in general lead to the need of additional time consuming drying steps which further increase the production costs.

In general, exploiting the press cake of lingonberry and other berries as a source of phenolic compounds is known in the art, the commercial interest having been mainly focused on obtaining extracts rich in anthocyanins and proanthocyanidins. There is a constant need to find plant sources, from which phenolic compounds could be easily isolated, and to develop new methods for enriching the most suitable active compounds from such sources to be used in specific applications. The present invention shows an option to make quercetin-rich extracts of natural berry origin (specifically lingonberry) for cosmetic applications.

### Summary of the Invention

The present invention relates to active cosmetic compositions which contain certain fractions of lingonberry (*Vaccinium vitis*-*idaea*) extracts obtained from lingonberry press cake, specifically from its dried fibrous part (lingonberry fiber powder). The invention also concerns a simple fractionation method for obtaining said fractions using only ethanol and ethyl acetate as extracting solvents. The present invention is thus directed to cosmetic compositions which contain common cosmetically acceptable substances and, in addition, new types of fractions of lingonberry extracts, containing phenolic compounds, especially quercetin in enriched form. The compositions of the invention thus contain at least one fraction obtained from dried lingonberry fiber powder. The invention also relates to the use of such compositions as, e.g., emulsion creams, such as day creams and foundation creams, but also lipsticks, skin serums and hair care products.

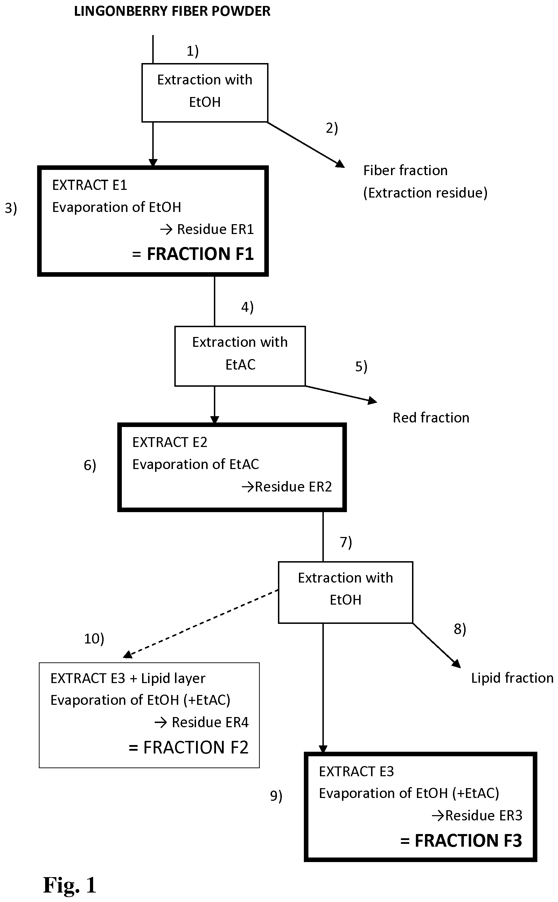

The fractionation method is based on three extractions in sequence (Figure 1), the extraction steps being carried out successively with ethanol, ethyl acetate and ethanol. Consequently, lingonberry fiber powder is first extracted by ethanol (EtOH). After evaporation the residue may be separated to two fractions by the second extraction with ethyl acetate (EtAC). After a few intermediate steps another ethanol extraction yields two additional fractions. All of the finished fractions include very low amounts of the solvents used.

### Brief Description of the Drawings

**Figure 1****.** Essential process steps of the fractionation.
**Figure 2****.** Chromatograms of lingonberry fiber powder fractions F1 - F3 analyzed by HPLC-DAD at 360 nm.

### Detailed Description of the Invention

The dried fibrous part of lingonberry press residue without seeds was shown by the present inventors to be a good raw material for making fractions containing lingonberry phenolic compounds. Lingonberry is a commercially important wild berry and lingonberry juice is an appreciated food use of lingonberries. Hence potential lingonberry press residue is easily available to be utilized in new non-food applications. As a food grade side stream it is readily available as a clean and safe raw material for making ingredients for, e.g., cosmetic applications where ingredients of natural origin are appreciated.

As indicated above, many organic solvents can be used to extract and fractionate phenolic compounds from berries. The compositions of the extracts and fractions highly depend on the solvents and processes used. Instead of using harmful solvents or, for instance, laborious and cost increasing purification or drying steps the inventors disclose here a new simple fractionation method which, in addition to small scale, can also easily be scaled up into a bigger scale for commercial production, without unnecessary high investment costs.

In this new method only ethanol and ethyl acetate are utilized in extractions. These organic solvents are regarded so safe that no maximum residual limits in food formulations were set by the directive 2009/32/EC of the European Parliament and of the Council. Ethanol and ethyl acetate have been included in the lists of possible solvents in some previously disclosed extraction methods for phenolic compounds. However, in the present invention these solvents are utilized undiluted and in a new manner.

Consequently, in the present invention side streams of berry industry, especially the press cake obtained when pressing juice from lingonberry (*Vaccinium vitis*-*idaea*), are exploited in producing ingredients which are useful in cosmetic compositions. The inventors have now found that certain fractions of lingonberry extract, which are prepared according to this invention, are especially advantageous for inclusion in cosmetic compositions. It was shown that the fractions used have high oxygen radical absorbance capacity (ORAC) and beneficial antioxidant effects when used in cosmetic compositions. It was shown that the quercetin found in these fractions appears mainly as an aglycon. The fractions showed a clear and concentration-dependent protective effect on keratinocytes against UV-induced H₂O₂ production. Also, the fractions were shown to have anti-ageing effects, i.e. they show inhibitory effects on elastase activity and elastase release by polymorphonuclear leukocytes (PMN). Furthermore, the effect of the fractions on expression of selected genes upon stress in keratinocytes was studied. It was shown that the fractions promote gene repair mechanism upon hydrogen peroxide-induced oxidative stress. Fraction F3 as described below was also shown to have a protective role against type II collagen degradation.

Whole lingonberries or juice can be used in making extracts containing lingonberry phenolic compounds, but the present invention utilizes the press cake. Dried lingonberry press cake, from which the seeds have been removed, was found to be a good source of useful compounds for cosmetic applications. For the purposes of this invention this raw material is called lingonberry fiber powder. The dried fiber-rich berry skin material contains significant amounts of phenolic compounds of the original berry in concentrated form.

Compared to the whole berries the press cake and the fiber powder made of it contain less sugars and organic acids, which mostly follow the juice in pressing. Hence complicated purification steps using e.g. resin columns, which are included in many existing methods to fractionate and recover phenolic compounds, are not needed when using dried lingonberry fiber powder as a raw material in the method of the present invention.

For the purposes of this invention it is advantageous to obtain fractions which contain compounds having antioxidant activities. Such fractions contain phenolic compounds in general, and quercetin in particular. In the method of the invention different quercetin-rich extracts and fractions are obtained from lingonberry fiber powder. The fractions are utilized as cosmetic ingredients according to the present invention. The concentrations of total phenolic compounds, as well as the quercetin concentration, and antioxidant capacities (ORAC) of the fractions of main interest are high.

Essential features in the fractionation process of the invention are sequential extractions and subsequent solvent evaporations and, between these steps, simple separations of the fractions formed. Dry lingonberry fiber powder is used as a raw material and only undiluted ethanol and ethyl acetate are used in extractions. Low water content decreases evaporation costs as such, and by promoting solvent recycling. It also ensures the use of simple methods to separate the fractions formed in the intermediate steps. The aim of the present invention is to produce quercetin-rich fractions instead of high purity quercetin.

Drying is important in exploiting the berry press cake in general, as well as according to the method disclosed here. Drying helps to maintain the activity of phenolic compounds and provides advantages in logistic and processing efficiency. Methods to make lingonberry fiber powders are currently in use and such powders are commercially available.

Quercetin occurs as various glucosides in lingonberries, but interestingly the inventors found out that quercetin as an aglygone appears to be the main flavonol compound in fractions made of lingonberry fiber powder. Most probably quercetin is released from its glucosides during the enzyme treatment before lingonberry juice pressing. It is a common practice to use pectinolytic enzymes to break down rigid structures of the berries in order to increase the juice yield. In general, aglycons of phenolic compounds show higher antioxidative activity compared to their glucosides. Hence the lingonberry fiber powder is an outstanding raw material in preparing fractions of high activity for cosmetic compositions.

### Essential process steps in the method of the present invention are (Figure 1):

1) Lingonberry fiber powder is extracted with ethanol (EtOH).
2) Extract E1 is separated from the extraction residue (fiber fraction).
3) EtOH is evaporated from E1; residue ER1 is Fraction **F1.**
4) ER1 is extracted with ethyl acetate (EtAC).
5) Extract E2 is separated from the red fraction.
6) EtAC is evaporated from E2; residue ER2 is obtained.
7) ER2 is extracted with EtOH.
8) Extract E3 is separated from the lipid layer.
9) EtOH and remnants of EtAC are evaporated from E3; residue ER3 is Fraction **F3.**
10) An alternative procedure to steps 8-9 is evaporation of EtOH and remnants of EtAC without removing the lipid layer. The resulting residue ER4 is Fraction **F2.**

The first process step (1) comprises extraction of the lingonberry fiber powder with ethanol in which the quercetin and other flavonols are well extracted but e.g. anthocyanins or proanthocyanidins to a lesser extent. The extraction is advantageously carried out using ultrasonication but other extraction methods currently available may also be used. When the fiber-containing solid extraction residue is separated (2), extract E1 is obtained. After ethanol is evaporated from E1 a paste like evaporation residue ER1 remains (3). It is called Fraction F1, which is useful in the cosmetic compositions of the present invention.

Fraction F1 is obtained using selective extraction by ethanol to favor enrichment of quercetin, but it also contains to some extent other ethanol soluble compounds e.g. red phenolic compounds (like anthocyanins and proanthocyanidins) and lipids (like fats, oils and waxes). Fraction F1 can still be fractionated to obtain new fractions, in which the relative quercetin concentrations are further increased.

In the second extraction Fraction F1 is extracted with ethyl acetate (4). Quercetin is well soluble in ethyl acetate, whereas most of the red phenolic compounds of Fraction F1 begin to separate from the solution. It is left standing without agitation after a short gentle mixing, and a distinct red fraction soon starts to settle on the bottom of the extraction vessel. Hence simple means to separate extract E2 from the top of the red fraction can be used (5). Possible methods like suction, decanting or filtering are applicable at this process step depending on, e.g., the settling time and instrumentation available.

Ethyl acetate is removed from E2 by evaporation (6). Ethyl acetate is evaporated easily, but small amounts tend to remain in residue ER2. Ethyl acetate is considered safe enough so that maximum residual limits have not been set e.g. for food applications (2009/32/EC). However, ethyl acetate odor even in low concentrations is very pungent and therefore ER2 is not useful in cosmetic applications. Complete evaporation of ethyl acetate remnants at this process step is not necessary (as shown below) but would only increase the evaporation time and costs.

The third extraction (7) is carried out by ethanol to remove ethyl acetate remnants from ER2 but again a simple fractionation method is provided to get new fractions. When ER2 is extracted with ethanol a lipid layer forms and settles on the bottom. The lipid layer, which has a distinct interface with the upper ethanol layer, is formed even more easily than the red fraction during the previous extraction step. Simple methods like suction or decanting are applicable to separate the extract E3 from the lipid fraction (8). After ethanol and ethyl acetate remnants are evaporated from the extract E3 (9) the solvent-free evaporation residue ER3, i.e. the fraction F3 is obtained. Fraction F3 is useful in cosmetic compositions.

As an alternative to process steps to obtain fraction F3, the third extraction with ethanol may be used to only remove the ethyl acetate remnants from ER2. In this case ethanol extraction is made to ER2 but the forming lipid layer is not separated from the extract E3 but it is left to mix in the residue during evaporation of the solvents (10). Solvent-free evaporation residue ER4 is obtained, and for the purposes of this invention it is called fraction F2, as its quercetin content is between the final fractions F1 and F3. All of the finished fractions include very low amounts of the solvents used.

Quercetin and total phenolic content in addition to oxygen radical absorbance capacity (ORAC) were measured from the main fractions (F1, F2, F3; Figure 1) and from the side fractions (red fraction and lipid fraction; Figure 1) prepared according to the method of the present invention (Examples 1 and 2). Both quercetin concentrations and ORAC values are increased in the main fractions in the order F1<F2<F3. Compared to F1, fraction F2 is concentrated in respect of quercetin as the red fraction is removed. Likewise, fraction F3 has the highest quercetin concentration compared to the other two fractions, because both the red fraction and the lipid fraction are removed.

The compounds of the red fraction (mainly anthocyanins and proanthocyanidins) are known antioxidants and the ORAC value of the red fraction is fairly high. However, when the red fraction is removed from fraction F1 the antioxidative capacity is increased due to enrichment of quercetin and possible other active compounds in fractions F2 and F3. The red compounds in large quantities are not suitable in formulations where strong red color is not wanted or in applications where their color is not stable. Depending on e.g. how accurately the separation steps are performed, some quercetin may follow the side fractions. The lipid fraction is practically free of quercetin and antioxidative capacity (Example 2).

In the process of the present invention optimization of the parameters in any of the process steps can be made, depending on the wanted properties of the resulting fractions. For instance, the ratio of ethyl acetate to evaporation residue ER1 in extraction step 4 influences the composition of fractions F2 and F3 in respect of type and quantity of the red phenolic compounds. The more ethyl acetate is used the more red fraction is formed. High amounts of ethyl acetate and accurate separation of extract E2 in step 5 provides fractions F2 and F3 which are practically free of red phenolic compounds. However, for the purposes of the present invention only a modest amount of ethyl acetate is needed as is shown in Example 1. It is seen as an advantage that quercetin-rich fractions also contain to some extent red phenolics and other compounds from the diversified array of berry phenolics, which probably together have synergy in biological protection of original berries and as an ingredient in cosmetic compositions.

The cosmetic composition according to the present invention can be, for example, an emulsion cream, such as day cream or foundation cream; a lipstick; a skin serum or a hair cosmetics product, such as a composition for hair or scalp care. Fractions F1 and F3 were tested and found to give advantageous properties to such compositions. Specifically, the quercetin-rich fraction F3 may replace chemically produced quercetin in such cosmetic compositions. The expression "Lingonberry Fractions F1, F2, (and) F3" as used below is intended to cover the three main quercetin-rich fractions obtained from the extraction process according to the present invention.

A cosmetic composition according to the invention contains, depending on the intended use of the composition, at least one of the Lingonberry Fractions F1, F2 and F3 in an amount of 0.001 to 25 % by weight, more preferably the amount of the fractions is 0.01 to 5 % by weight, and most preferably 0.01 to 1 % by weight.

The cosmetic composition according to the invention may also contain, depending on the intended use of the composition, lingonberry seed oil in an amount of 0.1 to 25 % by weight, more preferably the seed oil content is 0.1 to 5 % by weight, and most preferably 0.5 to 3 % by weight.

Additionally, the cosmetic composition according to the invention may also contain, depending on the intended use of the composition, different caring agents, e.g. peptides. These caring agents are typically in the amount of 0.1 to 40 % by weight, more preferably the caring agent content of a product is 0.1 to 20 % by weight, and most preferably 1 to 5 % by weight.

The emulsion cream according to the invention containing at least one of the Lingonberry Fractions F1, F2 and F3 can be of the type oil-in-water emulsion, water-in-oil emulsion, water-oil-water emulsion or a microemulsion. The emulsion cream composition according to the invention contains at least one of the Lingonberry Fractions F1, F2 and F3 in an amount of preferably 0.001 to 25 % by weight, more preferably 0.01 to 5 % by weight and most preferably 0.01 to 1 % by weight. The content of lingonberry seed oil in an emulsion cream may be in an amount of 0.1 to 25 % by weight. Preferably the seed oil content is 0.1 to 5 % by weight and most preferably 0.5 to 3 % by weight.

The emulsion cream compositions according to the invention contain, in addition, one or more adjuvants acceptable in the field of cosmetics, such as preservation agents, thickening agents, moisturizing agents, and other suitable additives, such as for example perfumes and/or colouring agents. Suitable preservation agents are for example parabens, sodium benzoate and phenoxyethanol. These preservation agents can be used alone or combined with each other. As thickening agent any thickening agent suitable in the field of cosmetics can be used, as long as it is compatible with the other components of the composition, for example xanthan gum and hydroxyethylcellulose, hydroxypropylmethylcellulose, Sclerotium gum, Chondrus Crispus, polyacrylates, polyacrylamides, cetearyl dimethicone crosspolymer and magnesium aluminium silicate. Thickening agents can be used alone or in combination with each other. Suitable moisturizing agents are for example heptyl undecylenate, hyaluronic acid or sodium PCA. In the emulsion creams according to the invention, also different skin conditioning agents, like for example tocopheryl acetate, and ethylhexylglycerin may be used.

In addition, one or more compounds acting as an emulsifier, i.e. a compound dispersing and stabilizing the oil in water is needed in the emulsion cream composition. Useful emulsifiers are all non-ionic emulsifiers accepted by the cosmetic legislation, such as, for example, glyceryl stearate, PEG-5 glyceryl stearate, PEG-1 00 stearate, PEG-30 dipolyhydroxystearate, lecithin, hydrogenated lecithine and PEG-8 bees wax, steareth-21, steareth-2, sorbitan olivate as well as a mixture of a fatty glucoside, such as for example cetearyl, cocoyl, or myristyl glucoside and a fatty alcohol, such as for example cetearyl, cetyl, stearyl, octyldodecanol, caprylic/capric triglyceride or myristyl alcohol. In addition, of anionic emulsifiers, for example stearic acid, sodium hydroxide and triethanolamine are useful. Also different chelating agents, like disodium EDTA and citric acid may be used.

The lipstick compositions according to the invention contain at least one of the Lingonberry Fractions F1, F2 and F3 in an amount of preferably 0.001 to 10 % by weight, more preferably 0.01 to 5 % by weight and most preferably 0.01 to 1 % by weight. In addition, these compositions may contain lingonberry seed oil in an amount of 0.01 to 10 % by weight. Preferably the seed oil content is 0.1 to 5 % by weight and most preferably 0.5 to 3 % by weight.

The lipstick compositions according to the invention contain in addition to at least one of the Lingonberry Fractions F1, F2 and F3 also other substances, like different waxes, oils, colouring and pearlescent agents, which are acceptable in the field of cosmetics and which are traditional components of lipstick compositions. Usable waxes are the natural waxes, such as bees wax, candelilla, carnauba, cereal based waxes, jojoba wax and their derivatives, in addition paraffin waxes and synthetic polyethylenes. Lipstick compositions according to the invention can also contain emulsifiers like PEG-8, behenyl alcohol and arachidyl alcohol, and various vitamins and derivates of those, like tocopherol and ascorbyl palmitate.

The lipstick compositions according to the invention can in addition contain one or more adjuvants and/or additives acceptable in the field of cosmetics, such as preservation agent. Suitable preservation agents are for example parabens. These preservation agents can be used alone or in combination with each other.

The compositions according to the invention intended for hair or scalp care contain at least one of the Lingonberry Fractions F1, F2 and F3 in an amount of preferably 0.001 to 10 % by weight, more preferably 0.001 to 5 % by weight and most preferably 0.001 to 1 % by weight. These compositions may also contain lingonberry seed oil in an amount of 0.01 to 10 % by weight. Preferably the seed oil content is 0.1 to 5 % by weight and most preferably 0.1 to 2 % by weight. Further, the compositions according to the invention intended for hair or scalp care may also contain different caring agents, e.g. juniper sprout extract. These caring agents are typically in the amount of 0.1 to 40 % by weight. Preferably the caring agent content of a product is 0.1 to 20 % by weight.

The compositions according to the invention intended for hair or scalp care contain in addition to at least one of the Lingonberry Fractions F1, F2 and F3 also other substances acceptable in the field of cosmetics, like cationically active substances, such as cetrimonium chloride, in addition an emulsion forming substance, such as for example cetyl alcohol, cetearyl alcohol, ceteareth-20. In addition, the compositions intended for hair or scalp care can contain one or more adjuvant acceptable in the field of cosmetics, such as a cellulose derivative, ethanol and/or water. Also oils, waxes and fatty alcohols can be present in the compositions according to the invention intended for hair or scalp care.

The skin serums according to the invention have the molecular ability to penetrate the skin deep within the layers of the skin and deposit the nutrients where they will be needed.

The serum compositions according to the invention contain at least one of the Lingonberry Fractions F1, F2 and F3 in an amount of preferably 0.001 to 25 % by weight, more preferably 0.01 to 5 % by weight and most preferably 0.01 to 1 % by weight. The serum compositions may further contain lingonberry seed oil in an amount of 0.01 to 25 % by weight. Preferably the seed oil content is 0.1 to 5 % by weight and most preferably 0.5 to 3 % by weight.

The serum compositions according to the invention typically contain also other substances acceptable in the field of cosmetics such as emulsifying agents, chelating agents, solvents, preservatives, stabilizers together with substances affecting the skin permeability of the composition. These substances may be e.g. methylpropanediol, glycerin, phenoxyethanol, hydroxyethyl acrylate/sodium acryloyldimethyl taurate copolymer, xanthan gum, propanediol, ammonium acryloyldimethyltaurate/VP copolymer, polyisobutene, disodium EDTA, lecithin, glucose, hydrogenated phosphatidylcholine, inulin lauryl carbamate, PEG-7 trimethylolpropane coconut ether, chondrus crispus, ethyl pyrrolidone, and cellulose gum. Additionally, the serum compositions according to the invention contain different moisturizing and skin conditioning agents such as heptyl undecylenate, ethylhexylglycerin, caprylyl glycol, ethylhexyl cocoate, peat extract and glycolipids.

The following examples further illustrate the invention.

### Example 1

### Preparation of fractions F1 and F3 from lingonberry fiber powder

400 g of lingonberry fiber powder (Kiantama Oy, Suomussalmi, Finland) was extracted with four litres of ethanol (ETAX A, Altia Oyj, Rajamäki, Finland) in ultrasonic bath (Grant Ultrasonic bath XB22, Grant Instruments (Cambridge) Ltd, Shepreth, England, 38 kHz,) for 3 x 20 min, stirring the mixture between ultra sound treatments. The fibrous extraction residue was separated by filter paper (5 µm) in Buchner funnel (Vacuum pump, MZ 2C+2AK, Vacuubrand GMBH+CO KG, Wertheim, Germany) to obtain extract **E1.**

Ethanol was evaporated from E1 in Rotavapor (R120 prof V, Buchi Labortechnic AG, Zürich, Switzerland) at 45°C, min 70 mbar. Ca. 90 g of dark red viscous paste-like evaporation residue ER1 was obtained. It was designated as Fraction **F1** ("Lingonberry Fraction F1") ready to be used in cosmetic compositions as such.

Fraction F1 was then extracted with 400 ml of ethyl acetate (EMSURE®, Merck KG aA, Darmstadt, Germany). The red fraction was separated by filter paper (5 µm) in Büchner funnel to obtain Extract **E2.** The ethyl acetate was evaporated from E2 in Rotavapor (45°C, min 70 mbar), whereby the evaporation residue **ER2** was obtained.

ER2 was extracted with 300 ml of ethanol. The lipid fraction was separated by decanting to obtain Extract **E3.** When ethanol was evaporated from E3 in Rotavapor (45°C, min 70 mbar) ca. 18 g of orange red evaporation residue **ER3** was obtained. It was designated as Fraction **F3** ("Lingonberry Fraction F3"), ready to be used in cosmetic compositions as such.

### Example 2

### Analyses of the fractions

Quercetin and total phenolic content in addition to oxygen radical absorbance capacity (ORAC) were measured from the fractions (F1 and F3) prepared in Example 1. Small amount of solvent-free fraction **F2** ("Lingonberry Fraction F2") and the side fractions (red and lipid fraction) were prepared for analysis using ethanol extraction as shown in process step 10 (Figure 1).

Quercetin analysis was performed by liquid chromatography method using an Agilent 1100 instrument (Agilent Technologies, Inc.) equipped with a photodiode array detector (DAD). The mobile phase consisted of eluents A) 1% formic acid in water (v/v) and B) 1% formic acid in acetonitrile, and the following linear gradient was run: 5-30% B at a flow rate of 0.2 mL/min. The column (200 × 2.00 mm, 5 µm, C18, 120 Å, HyperClone ODS; Phenomenex) temperature was 30°C. Quercetin content was monitored at 360 nm and UV spectra were obtained by scanning from 190 to 600 nm. Total phenolic content was analyzed by spectrophotometric Folin-Ciocalteu method (Magalhães *et al.,* 2010). Oxygen radical absorbing capacity (ORAC) analysis was performed by the method of Huang *et al.,* 2002, where the results were calculated as trolox equivalents (TE).

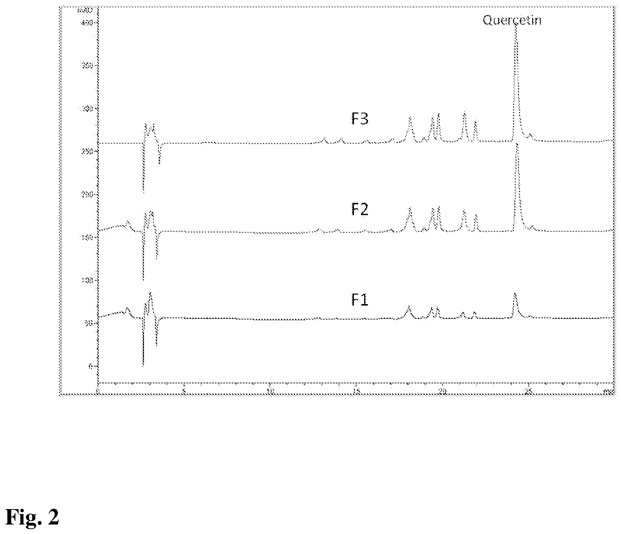

Quercetin content is increased in the main fractions in the order F1< F2<F3 (Figure 2). The highest quercetin concentration, 4.2 mg/g, in fraction F3 is about ten-fold higher than in the original lingonberry fiber powder (0.4 mg/g). Fractions F1 and F2 contained also high content of quercetin, 1.5 mg/g and 2.4 mg/g, respectively. The red fraction and the lipid fraction contained less quercetin, 0.9 mg/g and 0.3 mg/g, respectively.

Antioxidant capacities (ORAC) of the main fractions correlated well with the quercetin concentrations: Fraction F3 had the highest ORAC value (750 µmol TE/g) and F1 the lowest value (290 µmol TE/g), whereas the antioxidative capacity of Fraction F2 (430 µmol TE/g) was between the values of F1 and F3. The ORAC value of the red fraction was comparatively high (490 µmol TE/g), whereas the ORAC value for the lipid fraction was very low (30 µmol TE/g).

Total phenolic content, analysed by Folin-Ciocalteu method, supported the quercetin and ORAC results of the main fractions: Fraction F3 had the highest content (28 mg/g), whereas Fractions F1 and F2 were quite equal, 21 mg/g and 19 mg/g, respectively. The total phenolic content in the red fraction (29 µmol TE/g) was comparative to Fraction F3, although the ORAC value of Fraction F3 was much higher than that of the red fraction. This indicates that compounds enriched in Fraction F3 were more active in antioxidant capacity measurement than compounds in the red fraction. The content of total phenolics in the lipid fraction was low (9 µmol TE/g).

### Example 3

### Evaluation of antioxidant effect

The potential antioxidant effects of Fractions F1 and F3 were evaluated *in vitro* by measuring hydrogen peroxide production in normal human keratinocytes irradiated with UVA+B using a specific fluorescent probe (dHR, dihydrorhodamine). Keratinocytes were seeded in culture medium and incubated for 48 hours. The culture medium was then replaced by assay medium and the cells were further incubated for 24 hours. Then the cells were incubated in the presence of the specific fluorescent probe for the measure of hydrogen peroxide (dHR) for 10 minutes before adding or not (controls) the test compounds (F1 or F3) or the reference (BHA, 100 µM). The cells were pre-incubated for 45 minutes and then irradiated or not (non-irradiated control) with UVA+B (UVA 2.6 J/cm² + UVB 180 mJ/cm²). After irradiation, the cells were incubated for 30 minutes before flow cytometry analysis. A control without probe was performed in parallel and all experimental conditions were performed in n=3. The fluorescent intensity of the dHR probe, expressed in arbitrary unit (AU), is proportional to H₂O₂ production. The acquisition was performed by flow cytometry on 10,000 events for each replicate using a BD FACSarray™ Bioanalyzer (Becton-Dickinson).

Irradiation of keratinocytes with 180 mJ/cm² of UVB clearly increased H₂O₂ production and the treatment with the reference BHA at 100 µM resulted in a significant protection of cells from UV-induced H₂O₂ production (63% of protection). These effects were expected and validated the assay.

Fraction F3, tested from 0.01 to 0.3 mg/ml (diluted with ethanol), showed a strong, clear and concentration-dependent protective effect on keratinocytes against UV-induced H₂O₂ production (48%, 89% and 124% of protection at 0.03, 0.1 and 0.3 mg/ml, respectively). Fraction F1 also showed a protective effect against UV-induced H₂O₂ production. However, this effect was only observed at the highest test concentration of 0.3 mg/ml (91% of protection).

### Example 4

### Evaluation of the inhibitory effects on elastase activity and elastase release

Anti-ageing effects of Fractions F1 and F3 were studied by measuring the enzymatic activity of elastase, an enzyme responsible for elastin degradation. Elastin, a protein of the connective tissue (i.e. the extracellular matrix), is an elastic fiber which allows the skin to return to its original position after stretching.

The effects of the fractions were assessed by performing a biochemical assay on elastase activity and a cellular assay on elastase release by neutrophils (also called polymorphonuclear leukocytes or PMN). PMN, the most common type of granulocytes, phagocytize and destroy microorganisms, e.g. bacteria, and thus play a key role in innate immunity against bacterial infection. Elastase stored in the intracellular granules of activated PMN is an essential enzyme for the degradation of foreign proteins during phagocytosis. Once released from PMN, elastase can cause severe damage to macromolecules in cell membranes and the extracellular space, including proteoglycans, elastin and fibronectin.

Peptide formyl-methionyl-leucyl-phenylalanine (fMLP) was used in association with cytochalasin B, a cell-permeable mycotoxin, to stimulate elastase release by neutrophils. Quercetin, a flavonoid presenting immunomodulatory properties by inhibiting enzymes involved in the production of inflammatory molecules, was used as a comparative molecule since it was previously described as an inhibitory molecule by Kanashiro *et al*.,2007 on elastase release by PMN stimulated with (fMLP+CB). The concentrations for quercetin in the present study were selected in order to match the concentration tested in the publication by Kanashiro *et al.,* 2007.

### Elastase activity quantification:

The test compounds or reference (AAPV tested at 0.1 mM) were pre-incubated for 10 minutes at room temperature (RT) in the presence of the enzyme (human leukocyte elastase: EC 3.4.21.37).The fluorescent substrate (DQ™-elastin) was then added and the mixtures were incubated for 1 hour at RT. A compound interference control in the presence of reactive medium without enzyme (autofluorescence measure) and a "quenching" control (eventual fluorescence absorption by the compound) were carried out in parallel. All experimental conditions were performed in n=3 except for interference and "quenching" controls, which were performed in n=2. At the end of incubation, fluorescence produced following the substrate degradation was measured by fluorometry (λex 485 nm, λem 538 nm, SpectroMax Gemini, Molecular Devices®). The results were expressed in inhibition percentage of enzymatic activity.

The reference compound AAPV, tested at 0.1 mM, highly inhibited leukocyte elastase activity (95% of inhibition). This effect was expected and validated this assay. Fraction F3 tested at 0.3 and 1 mg/ml (diluted in ethanol) presented a significant concentration-dependent inhibitory effect on human leukocyte elastase activity reaching 72% of inhibition at the highest concentration. Fraction F1 tested at 0.3 and 1 mg/ml (diluted in ethanol) also presented a significant concentration-dependent inhibitory effect on human leukocyte elastase activity (66% of inhibition at the highest concentration).

### Elastase release by stimulated PMN followed by elastase activity measurement:

PMN, isolated from human peripheral blood (22-year-old male donor), were pre-incubated for 10 minutes in assay medium containing or not (controls) the test fractions F1 and F3 in the presence or not (non-stimulated control) of cytochalasin B (1µM). Degranulation was then triggered by adding fMLP (1µM) and the cells were incubated for 30 minutes. All the experimental conditions were performed in n=3, except for controls which were performed in n=6. At the end of the incubation, cell supernatants were collected in order to quantify the activity of the released elastase and thus to estimate indirectly the amount of released elastase.

The elastase peptide substrate *N*-succinyl-Ala-Ala-Val-*p*-nitroanilide (SAAVNA, 1 mM) was added to all supernatants and the release of the yellow product *p*-NA was quantified by photometry at 405 nm after substrate addition (TO) and after 20 hours of incubation. The results were expressed in inhibition percentage of enzymatic activity.

Treatment with the association of cytochalasin B and fMLP highly induced elastase release by PMN. This result was expected and validated the assay. Fraction F3, tested at 0.03, 0.1 and 0.3 mg/ml (diluted in ethanol) showed a concentration-dependent inhibitory effect on elastase release by PMN stimulated with CB+fMLP (17, 27 and 45% of inhibition). A concentration-dependent inhibitory effect on elastase release was also observed in the presence of the two highest test concentrations of Fraction F1 (13 and 37% of inhibition at 0.1 and 0.3 mg/ml, respectively). Quercetin only showed a weak inhibitory effect on elastase release by stimulated PMN (7 and 13% of inhibition when tested at 3 and 10 µM).

### Example 5

### Effects on gene expression changes upon oxidative stress in keratinocytes

The effects of Fractions F1 and F3 on gene expression changes upon oxidative stress in keratinocytes were studied. The first part of the study consisted of testing the solubility of Fractions F1 and F3 and evaluating their effects on the morphology of human keratinocytes. The optimal concentration of the compounds that was tolerated by the cells was determined. The selected concentrations were 0.125 mg/ml for F1 and 0.05 mg/ml for F3. HPEK cells (normal human epidermal keratinocyte progenitor cells) were grown in epidermal keratinocyte medium containing supplements under 5% CO₂ at 37°C for 24 hours. Each sample was prepared in duplicate. Fractions F1 and F3 were then added on the cells and the cells were grown further for 24 h in the presence of the fractions before they were either treated with 500 µM H₂O₂ for 30 min to induce oxidative stress or left untreated (H₂O₂ concentration was chosen based on induction of ROS and the level of changes in gene expression). Cells that were treated with H₂O₂ were washed before and after H₂O₂ treatment which was done in cell culture medium without supplements. After washing, treated and untreated wells received fresh medium containing Fractions F1 and F3. The cells were further cultured for 6 h to allow induction of gene expression. Following RNA isolation and preparation of cDNA, the messenger RNA levels of the selected genes were determined using quantitative Taqman PCR. The expression levels of all genes were normalized to the expression of β-actin which is a commonly used reference gene for RNA analyses. The final results consist of the average gene expression levels from two independent PCR runs.

Sirtuin 1(SIRT1) is involved in repair of DNA damage caused by oxidative stress. Increased SIRT1 expression promotes survival and suppresses age-dependent changes in a mouse model. Both F1 and F3 increased SIRT1 expression upon oxidative stress. In other words; F1 and F3 were shown to promote gene repair mechanism upon hydrogen peroxide-induced oxidative stress.

Growth arrest and DNA damage-inducible, alpha (GADD45A) is a gene that is induced following stressful growth arrest conditions and DNA damage. In cells the levels of GADD45A should be increased upon oxidative stress. In this study the GADD45A expression was induced in response to oxidative stress and the increase was further potentiated by F1 and F3. In other words; F1 and F3 were shown to promote gene repair mechanism upon hydrogen peroxide-induced oxidative stress.

Thioredoxin (TXN) is an antioxidant, growth stimulatory and anti-inflammatory protein. Oxidative stress induces the expression of TXN. Both F1 and F3 increased TXN expression upon oxidative stress. This means that F1 and F3 might have beneficial effects in keratinocytes under normal and stressful conditions, as thioredoxins are antioxidants that are known to reduce oxidative stress and thioredoxin overexpression has been linked to improved inflammation.

Matrix metalloproteinase 13 (MMP13) is a key proteinase in degradation of type II collagen. Oxidative stress induces the expression of MMP13. It was shown that F3 decreases MMP13 expression under normal conditions, thus F3 has a protective role against type II collagen degradation.

### Example 6

An example of ingredients for a ready-for-use emulsion cream (day cream) composition for facial and throat skin care containing lingonberry seed oil and at least one of the Lingonberry Fractions F1, F2 and F3 is shown in the following Table 1. The described emulsion cream composition moisturizes, brightens and softens the skin. In addition, the emulsion cream according to the invention possesses antioxidant properties.

**Table 1. Day Cream**

| INGREDIENTS | Ratio % |
|---|---|
| Aqua (Water) | Add 100 |
| Steareth-2 | 4.0-5.0 |
| Dimethicone | 3.0-5.0 |
| Glycerin | 2.0-5.0 |
| Steareth-21 | 2.500 |
| Octyldodecanol | 1.0-3.0 |
| Stearic Acid | 1.0-3.0 |
| Lingonberry Seed Oil | 0.1-5.0 |
| Lingonberry Fractions F1, F2, F3 | 0.1-5.0 |
| Cetearyl Alcohol | 1.500 |
| Heptyl Undecylenate | 1.0-2.0 |
| Caprylic/Capric Triglyceride | 1.000 |
| PEG-30 Dipolyhydroxystearate | 0.5-1.0 |
| Aluminum Starch Octenyl Succinate | 0.5-1.0 |
| Phenoxyethanol | 0.5-1.0 |
| Cetearyl Dimethicone Crosspolymer | 0.5-1.0 |
| Tocopheryl Acetate | 0.2-0.8 |
| PPG-15 Stearyl Ether | 0.3-0.6 |
| Parfum (Fragrance) | 0.390 |
| Lecithin | 0.203 |
| Xanthan Gum | 0.15-0.2 |
| Acrylates/C10-30 Alkyl Acrylate Crosspolymer | 0.100 |
| Disodium EDTA | 0.100 |
| Magnesium Ascorbyl Phosphate | 0.100 |

### Example 7

An example of ingredients for ready-for-use emulsion cream (day cream) composition containing at least one of the Lingonberry Fractions F1, F2 and F3 for facial and throat skin care is presented in the following Table 2. The emulsion cream composition improves the revitalization of the skin. In addition, it reduces, softens and smoothes fine lines in the skin. It has also antioxidant properties.

**Table 2. Day Cream**

| INGREDIENTS | Ratio % |
|---|---|
| Aqua (Water) | Add 100 |
| Steareth-2 | 4.0-5.0 |
| Dimethicone | 3.0-5.0 |
| Glycerin | 2.0-5.0 |
| Steareth-21 | 2.500 |
| Octyldodecanol | 1.0-3.0 |
| Stearic Acid | 1.0-3.0 |
| Lingonberry Fractions F1, F2, F3 | 0.01-5.0 |
| Cetearyl Alcohol | 1.500 |
| Heptyl Undecylenate | 1.0-2.0 |
| Caprylic/Capric Triglyceride | 1.000 |
| PEG-30 Dipolyhydroxystearate | 0.5-1.0 |
| Aluminum Starch Octenyl Succinate | 0.5-1.0 |
| Phenoxyethanol | 0.5-1.0 |
| Cetearyl Dimethicone Crosspolymer | 0.5-1.0 |
| Tocopheryl Acetate | 0.2-0.8 |
| PPG-15 Stearyl Ether | 0.3-0.6 |
| Parfum (Fragrance) | 0.390 |
| Lecithin | 0.203 |
| Xanthan Gum | 0.15-0.2 |
| Acrylates/C10-30 Alkyl Acrylate Crosspolymer | 0.100 |
| Disodium EDTA | 0.100 |
| Magnesium Ascorbyl Phosphate | 0.100 |

### Example 8

An example of ingredients for a foundation cream composition containing at least one of the Lingonberry Fractions F1, F2 and F3 for facial skin is presented in Table 3. The cream moisturizes the skin, and protects it from UV-radiation. The cream also gives the skin light coverage and colour.

**Table 3. Foundation Cream**

| INGREDIENTS | Ratio % |
|---|---|
| Aqua (Water) | 50.0-55.0 |
| Cyclopentasiloxane | 10.0-15.0 |
| (CI 77891) Titanium Dioxide | 5.0-10.0 |
| Cyclohexasiloxane | 3.0-5.0 |
| Isododecane | 2.0-10.0 |
| Ethylhexyl Methoxycinnamate (Octinoxate) | 1.0-6.0 |
| Polyglyceryl-4 isostearate | 1.0-2.0 |
| Magnesium Sulfate | 1.0-2.0 |
| Cetyl PEG/PPG-10/1 Dimethicone | 1.0-2.0 |
| Hexyl laurate | 1.0-2.0 |
| (CI 77491) Iron Oxides | 1.0-2.0 |
| MICA | 1.0-2.0 |
| Nylon-12 | 0.5-1.0 |
| Propylene Glycol | 0.5-1.0 |
| Titanium Dioxide | 0.5-1.0 |
| Phenoxyethanol | 0.5-1.0 |
| Disteardimonium Hectorite | 0.5-1.0 |
| Silica | 0.5-1.0 |
| (CI 77492) Iron Oxides | 0.1-1.0 |
| Butylene Glycol | 0.1-1.0 |
| Lingonberry Fractions F1, F2, F3 | 0.01-5.0 |
| (CI 77499) Iron Oxides | 0.1-1.0 |
| Perfluorooctyl Triethoxysilane | 0.1-1.0 |
| Propylene Carbonate | 0.05-0.3 |
| Dimethicone Crosspolymer | 0.05-0.3 |
| Methicone | 0.05-0.3 |
| Parfum (Fragrance) | 0.05-0.3 |
| Ethylhexylglycerin | 0.05-0.3 |
| Alumina | 0.05-0.3 |
| Triethoxycaprylylsilane | 0.05-0.3 |

### Example 9

An example of ingredients for a lipstick composition containing at least one of the Lingonberry Fractions F1, F2 and F3 is presented in Table 4. The lipstick composition according to the invention moisturizes the lips and provides lip care.

**Table 4. A lipstick composition.**

| INGREDIENTS | Ratio % |
|---|---|
| Ricinus communis Seed Oil | Add 100 |
| Squalane | 17.500 |
| Candelilla cera | 12.100 |
| Apricot kernel oil peg-6 esters | 8.000 |
| Acetylated lanolin | 5.900 |
| Aqua | 5.000 |
| Peg-8 beeswax | 4.700 |
| Hydrogenated polydecene | 4.000 |
| Butyrospermum parkii | 4.000 |
| Isopropyl myristate | 3.900 |
| Cera alba | 2.300 |
| Ethylcellulose | 2.000 |
| Lingonberry Fractions F1, F2, F3 | 0.01-2.0 |
| Polyethylene | 2.000 |
| Mica | 1.800 |
| Arachidyl alcohol | 1.400 |
| Behenyl alcohol | 1.300 |
| Arachidyl glucoside | 1.300 |
| Perfume | 1.000 |
| Lingonberry Seed Oil | 1.000 |
| Carnauba Wax | 0.900 |
| Propylparaben | 0.100 |
| PEG-8 | 0.100 |
| Tocopherol | 0.035 |
| Ascorbyl palmitate | 0.006 |
| Ascorbic Acid | 0.001 |
| Citric Acid | 0.001 |
| Colours | 4.800 |

### Example 10

Another example of a lipstick composition containing at least one of the Lingonberry Fractions F1, F2 and F3 and lingonberry seed oil is presented in Table 5.

**Table 5.** Another lipstick composition.

| INGREDIENTS | Ratio % |
|---|---|
| Ricinus communis Seed Oil | Add 100 |
| Oleyl alcohol | 20.000 |
| Candelilla cera | 9.800 |
| Cera alba | 5.700 |
| Acetylated lanolin | 4.700 |
| Isopropyl lanolate | 4.000 |
| Isopropyl myristate | 3.200 |
| Hydrogenated polydecene | 3.000 |
| Ethylhexyl methoxycinnamate | 2.000 |
| Mica | 1.800 |
| Microcrystalline wax | 1.600 |
| Lingonberry Seed Oil | 1.000 |
| Lingonberry Fractions F1, F2, F3 | 0.01-2.0 |
| Perfume | 1.000 |
| Carnauba | 0.700 |
| Propyl paraben | 0.090 |
| PEG-8 | 0.040 |
| Tocopherol | 0.020 |
| Ascorbyl palmitate | 0.003 |
| Ascorbic Acid | 0.001 |
| Citric Acid | 0.001 |
| Colours | 4.800 |

### Example 11

An example of ingredients for a facial serum product containing at least one of the Lingonberry Fractions F1, F2 and F3 is presented in Table 6.

**Table 6. A facial serum composition.**

| INGREDIENTS | Ratio % |
|---|---|
| Aqua (Water) | Add 100 |
| Heptyl Undecylenate | 4.000 |
| Methylpropanediol | 3.000 |
| Glycerin | 3.000 |
| Lingonberry Fractions F1, F2, F3 | 0.01-2.0 |
| Phenoxyethanol | 1.000 |
| Hydroxyethyl Acrylate/Sodium Acryloldimethyl Taurate Copolymer | 0.300 |
| Xanthan Gum | 0.1-0.4 |
| PVP | 0.200 |
| Ethylhexylglycerin | 0.200 |
| Propanediol | 0.163 |
| Ammonium Acryloyldimethyltaurate/ VP Copolymer | 0.150 |
| Caprylyl Glycol | 0.150 |
| Polyisobutene | 0.150 |
| Ethylhexyl Cocoate | 0.100 |
| Disodium EDTA | 0.100 |
| Lecithin | 0.056 |
| PEG-7 Trimethylolpropane Coconut Ether | 0,025 |
| Glucose | 0.008 |
| Peat Extract | 0.007 |
| Chondrus Crispus (Carrageenan) | 0.002 |
| Glycolipids | 0.000 |

### Example 12

An example of ingredients for another facial serum product containing at least one of the Lingonberry Fractions F1, F2 and F3 and lingonberry seed oil is shown in Table 7.

**Table 7. Another facial serum composition**

| INGREDIENTS | Ratio % |
|---|---|
| Aqua (Water) | Add 100 |
| Heptyl Undecylenate | 4.000 |
| Methylpropanediol | 3.000 |
| Glycerin | 3.000 |
| Phenoxyethanol | 1.000 |
| Lingonberry Seed Oil | 0.1-5.0 |
| Lingonberry Fractions F1, F2, F3 | 0.01-2.0 |
| Hydroxyethyl Acrylate/Sodium Acryloldimethyl Taurate Copolymer | 0.300 |
| Xanthan Gum | 0.266 |
| PVP | 0.200 |
| Ethylhexylglycerin | 0.200 |
| Propanediol | 0.163 |
| Ammonium Acryloyldimethyltaurate/ VP Copolymer | 0.150 |
| Caprylyl Glycol | 0.150 |
| Polyisobutene | 0.150 |
| Ethylhexyl Cocoate | 0.100 |
| Disodium EDTA | 0.100 |
| Lecithin | 0.056 |
| PEG-7 Trimethylolpropane Coconut Ether | 0.025 |
| Glucose | 0.008 |
| Peat Extract | 0.007 |
| Chondrus Crispus (Carrageenan) | 0.002 |

### Example 13

An example of a caring or conditioning product for hair. The composition containing at least one of the Lingonberry Fractions F1, F2 and F3 is shown in Table 8. Lingonberry seed oil can be used in caring products to impart gloss to the hair and to improve the manageability of the hair.

**Table 8. A caring or conditioning product composition for hair.**

| INGREDIENTS | Ratio % |
|---|---|
| Aqua (Water) | Add 100 |
| Lingonberry Fractions F1, F2, F3 | 0.01-2.0 |
| Emulsifier, e.g. ceteareth 20 | 0.1-10 |
| Oils, waxes and/or fatty alcohols | 0.1-10 |
| Thickeners, e.g. cellulose derivatives | 0.1-10 |
| Cationic surfactants, e.g. cetrimonium chloride | 0.1-15 |
| Caring oils (e.g. Lingonberry Seed Oil) | 0.1-20 |
| Moisturizers, e.g. sorbitol | 0.1-10 |
| Polymers | 0.1-10 |
| Preservation agent, pH regulating agents, Perfume, Colour | |

### Example 14

Another example of a caring or conditioning product for hair. The composition containing lingonberry seed oil and at least one of the Lingonberry Fractions F1, F2 and F3 is shown in Table 9. Lingonberry seed oil can be used in caring products to impart gloss to the hair and to improve the manageability of the hair.

**Table 9. Another caring or conditioning product composition for hair.**

| INGREDIENTS | Ratio % |
|---|---|
| Aqua (Water) | Add 100 |
| Lingonberry Seed Oil | 0.1-5.0 |
| Lingonberry Fractions F1, F2, F3 | 0.01-2.0 |
| Emulsifier, e.g. ceteareth 20 | 0.1-10 |
| Oils, waxes and/or fatty alcohols | 0.1-10 |
| Thickeners, e.g. cellulose derivatives | 0.1-10 |
| Cationic surfactants, e.g. cetrimonium chloride | 0.1-15 |
| Caring oils (e.g. isopropyl myristate) | 0.1-20 |
| Moisturizers, e.g. sorbitol | 0.1-10 |
| Polymers | 0.1-10 |
| Preservation agent, pH regulating agents, Perfume, Colour | |

### Example 15

An example of a caring product for scalp treatment. The composition containing at least one of the Lingonberry Fractions F1, F2 and F3 is shown in Table 10.

**Table 10. A caring product composition for scalp treatment.**

| INGREDIENTS | Ratio % |
|---|---|
| Aqua (Water) | Add 100 |
| Ethanol | 10-60 |
| Lingonberry Fractions F1, F2, F3 | 0.01-2.0 |
| Moisturizing agents, e.g. sorbitol | 0.1-10 |
| Caring agents, e.g. Juniper sprout extract | 0.1-20 |
| Preservation agent, pH regulating agents, Colour, Perfume | |

### Example 16

Another example of a caring product for scalp treatment. The composition containing lingonberry seed oil in combination with at least one of the Lingonberry Fractions F1, F2 and F3 is shown in Table 11. Products intended for special care are emulsions or creams which impart a caring and moisturizing effect to the scalp by means of the lingonberry seed oil.

**Table 11. Another caring product composition for scalp treatment**

| INGREDIENTS | Ratio % |
|---|---|
| Aqua (Water) | Add 100 |
| Ethanol | 10-60 |
| Lingonberry Seed Oil | 0.1-5.0 |
| Lingonberry Fractions F1, F2, F3 | 0.01-2.0 |
| Moisturizing agents, e.g. sorbitol | 0.1-10 |
| Caring agents, e.g. Juniper sprout extract | 0.1-20 |
| Preservation agent, pH regulating agents, Colour, Perfume | |

### References

Ek, S., Kartimo, H., Mattila, S. and Tolonen, A. Characterization of Phenolic Compounds from Lingonberry (Vaccinium vitis-idaea), J. Agric. Food Chem., 2006, 54, 9834-9842
Huang, D., Ou, B., Hampsch-Woodill, M., Flanagan, J.A. and Prior, R. L., High-Throughput assay of oxygen radical absorbance capacity (ORAC) using a multichannel liquid handling system coupled with a microplate fluorescence reader in 96-well format, J. Agric. Food Chem., 2002, 50, 4437-4444.
Kanashiro, A., Souza, J.G., Kabeya, L.M., Azzolini, A.E. and Lucisano-Valim, Y.M. Elastase release by stimulated neutrophils inhibited by flavonoids: importance of the catechol group, Z Naturforsch C., 2007, 62(5-6), 357-361.
Kylli, P., Berry phenolics: isolation, analysis, identification and antioxidant properties, Academic Dissertation, University of Helsinki, 2011.
Magalhães, L. M., Santos, F., Segundo, M.A., Reis, S. and Lima, J.L.F.C. Rapid microplate high-throughput methodology for assessment of Folin-Ciocalteu reducing capacity, Talanta, 2010, 83, 441-447.
USDA Database for the Flavonoid Content of Selected Foods, Release 3.0. Nutrient Data Laboratory Home Page: (http://www.ars.usda.gov/nutrientdata/flav).U.S. Department of Agriculture, Agricultural Research Service. 2011.

## Claims

1. A process for producing fractions of lingonberry extracts containing phenolic compounds, comprising the steps of:
a) extracting dried lingonberry fiber powder with ethanol to obtain a first extract (E1),
b) evaporating ethanol from the first extract E1 to obtain a first fraction (F1),
c) extracting the first fraction F1 with ethyl acetate to obtain a second extract (E2),
d) evaporating ethyl acetate from the second extract E2 to obtain an evaporation residue (ER2),
e) extracting the evaporation residue ER2 with ethanol to obtain a third extract (E3) together with a lipid layer, and
f) without removing the lipid layer, evaporating ethanol and the ethyl acetate remnants to obtain a second fraction (F2), or, alternatively,
g) separating the third extract E3 from the lipid layer, and
h) evaporating ethanol and the ethyl acetate remnants from the third extract E3 to obtain a third fraction (F3).

2. The process according to claim 1, wherein undiluted ethanol and ethyl acetate are used in the extractions.

3. The process according to claim 1 or 2, wherein the phenolic compound is quercetin.

4. A cosmetic composition comprising cosmetically acceptable substances and, in addition, two quercetin-rich fractions obtained from dried lingonberry fiber powder with the process according to any one of claims 1 to 3.

5. The cosmetic composition according to claim 4, wherein said two quercetin-rich fractions are fractions F1 and F3.

6. A method of cosmetically treating the skin of a human subject, comprising the step of topically applying to the skin a cosmetically effective amount of the cosmetic composition according to claim 4 or 5.

7. The method according to claim 6, wherein the cosmetic composition to be applied to the skin has anti-ageing effects.

8. The method according to claim 6, wherein the cosmetic composition to be applied to the skin has a protective role against type II collagen degradation.

9. The cosmetic composition according to claim 4 or 5 for use in a method for promoting gene repair mechanism upon hydrogen peroxide-induced oxidative stress.

## Patentansprüche

1. Verfahren zum Herstellen von Fraktionen von Preiselbeerextrakten, die Phenolverbindungen enthalten, umfassend die folgenden Schritte:
a) Extrahieren von getrocknetem Preiselbeerfaserpulver mit Ethanol, um einen ersten Extrakt (E1) zu gewinnen,
b) Verdampfen des Ethanols aus dem ersten Extrakt E1, um eine erste Fraktion (F1) zu gewinnen,
c) Extrahieren der ersten Fraktion F1 mit Ethylacetat, um einen zweiten Extrakt (E2) zu gewinnen,
d) Verdampfen des Ethylacetats aus dem zweiten Extrakt E2, um einen Verdampfungsrückstand (ER2) zu gewinnen,
e) Extrahieren des Verdampfungsrückstands ER2 mit Ethanol, um einen dritten Extrakt (E3) zusammen mit einer Lipidschicht zu gewinnen, und
f) Verdampfen der Ethanol- und Ethylacetatereste, ohne die Lipidschicht zu entfernen, um eine zweite Fraktion (F2) zu gewinnen, oder alternativ
g) Trennen des dritten Extrakts E3 von der Lipidschicht, und
h) Verdampfen der Ethanol- und Ethylacetatreste aus dem dritten Extrakt E3, um eine dritte Fraktion (F3) zu gewinnen.

2. Verfahren nach Anspruch 1, wobei bei den Extraktionen unverdünntes Ethanol und Ethylacetat verwendet werden.

3. Verfahren nach Anspruch 1 oder 2, wobei es sich bei der Phenolverbindung um Quercetin handelt.

4. Kosmetische Zusammensetzung, umfassend kosmetisch annehmbare Substanzen und zusätzlich zwei quercetinreiche Fraktionen, die mit dem Verfahren nach einem der Ansprüche 1 bis 3 aus getrocknetem Preiselbeerfaserpulver gewonnen wurden.

5. Kosmetische Zusammensetzung nach Anspruch 4, wobei es sich bei den zwei quercetinreichen Fraktionen um die Fraktionen F1 und F3 handelt.

6. Verfahren zum kosmetischen Behandeln der Haut einer menschlichen Person, umfassend den Schritt des topischen Auftragens einer kosmetisch wirksamen Menge der kosmetischen Zusammensetzung nach Anspruch 4 oder 5 auf die Haut.

7. Verfahren nach Anspruch 6, wobei die auf die Haut aufzutragende kosmetische Zusammensetzung Alterungsschutzeffekte aufweist.

8. Verfahren nach Anspruch 6, wobei die auf die Haut aufzutragende kosmetische Zusammensetzung eine schützende Wirkung gegen Typ-II-Collagen-Abbau aufweist.

9. Kosmetische Zusammensetzung nach Anspruch 4 oder 5 zur Verwendung bei einem Verfahren zur Förderung des Genreparaturmechanismus bei Wasserstoffperoxid-induziertem oxidativem Stress.

## Revendications

1. Processus pour produire des fractions d'extraits d'airelle rouge contenant des composés phénoliques, comprenant les étapes suivantes :
a) extraction d'une poudre de fibre d'airelle rouge séchée avec de l'éthanol pour obtenir un premier extrait (E1),
b) évaporation de l'éthanol depuis le premier extrait E1 pour obtenir une première fraction (F1),
c) extraction de la première fraction F1 avec de l'acétate d'éthyle pour obtenir un deuxième extrait (E2),
d) évaporation de l'acétate d'éthyle depuis le deuxième extrait E2 pour obtenir un résidu d'évaporation (ER2),
e) extraction du résidu d'évaporation ER2 avec de l'éthanol pour obtenir un troisième extrait (E3) en même temps qu'une couche de lipide, et
f) sans enlever la couche de lipide, évaporation des restes d'éthanol et d'acétate d'éthyle pour obtenir une deuxième fraction (F2), ou, comme variante,
g) séparation du troisième extrait E3 depuis la couche de lipide, et
h) évaporation des restes d'éthanol et d'acétate d'éthyle depuis le troisième extrait E3 pour obtenir une troisième fraction (F3).

2. Processus selon la revendication 1, dans lequel de l'éthanol et de l'acétate d'éthyle non dilués sont utilisés dans les extractions.

3. Processus selon la revendication 1 ou 2, dans lequel le composé phénolique est de la quercétine.

4. Composition cosmétique comprenant des substances acceptables d'un point de vue cosmétique et, de plus, deux fractions riches en quercétine obtenues à partir de poudre de fibre d'airelle rouge séchée avec le processus selon l'une quelconque des revendications 1 à 3.

5. Composition cosmétique selon la revendication 4, dans laquelle lesdites deux fractions riches en quercétine sont des fractions F1 et F3.

6. Procédé pour traiter de façon cosmétique la peau d'un sujet humain, comprenant l'étape d'application par voie topique à la peau d'une quantité efficace d'un point de vue cosmétique de la composition cosmétique selon la revendication 4 ou 5.

7. Procédé selon la revendication 6, dans lequel la composition cosmétique à appliquer à la peau a des effets anti-vieillissement.

8. Procédé selon la revendication 6, dans lequel la composition cosmétique à appliquer à la peau a un rôle protecteur contre la dégénérescence du collagène de type II.

9. Composition cosmétique selon la revendication 4 ou 5 à utiliser dans un procédé pour favoriser le mécanisme de réparation de gène sous un stress oxydatif induit par du peroxyde d'hydrogène.
